# EUROPEAN PATENT APPLICATION

(11) **EP 4 772 110 A1**
(43) Date of publication of application: **08.07.2026**
(21) Application number: 25150351.2
(22) Date of filing: 06.01.2025
(51) Int. Cl.: A61B 5/024, A61B 5/1455, A61B 5/00

(54) **OPTICAL BIOLOGICAL SIGNAL MEASUREMENT**

(71) Applicant: Koninklijke Philips N.V., 5656 AE Eindhoven (NL)
(72) Inventor: VAN DEN DUNGEN, Wilhelmus Andreas Marinus Arnoldus Maria, 5656 AG Eindhoven (NL); PAULUSSEN, Elvira Johanna Maria, 5656 AG Eindhoven (NL); DAMINK, Paulus Henricus Antonius, 5656 AG Eindhoven (NL)
(74) Representative: Philips Intellectual Property & Standards

(57) **Abstract**

A method for measuring biological signals, such as oxygen saturation levels in the blood, using optical sensors. The method is based on computing a biological signal using a plurality of optical signals, the plurality of optical signals acquired across multiple ray paths through the tissue. In some embodiments, the optical signals of the group are acquired simultaneously, and each optical signal in the group corresponds to a different spatial ray path through the tissue of the subject from at least one optical source to at least one optical detector.

## Description

### FIELD OF THE INVENTION

The present invention relates to systems and methods for measuring biological signals using optical interrogation of tissue.

### BACKGROUND OF THE INVENTION

Optical biological signal measurement is a widely used non-invasive method for monitoring various physiological parameters. One common example is photoplethysmography (PPG), which can be used to measure heart rate, blood oxygen saturation, and other vital signs. PPG sensors typically employ one or more light-emitting diodes (LEDs) that emit optical radiation (e.g. light) at different wavelengths, and one or more photodetectors to measure the amount of optical radiation that passes through or is reflected by the tissue. The variations in optical radiation absorption and scattering as blood flows through the tissue are used to derive physiological information.

In optical biological signal measurement, a key consideration is the distinction between functional and non-functional optical radiation. Functional optical radiation (also referred to as `intended optical radiation') refers to the portion of emitted optical radiation that interacts with the tissue in a way that provides useful information about the physiological parameter being measured. Non-functional optical radiation (also referred to as 'incidental optical radiation' or 'shunt optical radiation'), on the other hand, may include optical radiation that is scattered or absorbed by non-target tissues, or optical radiation that follows paths that do not capture the desired physiological information.

The balance between penetration depth and signal strength presents another challenge in optical biological signal measurement. As optical radiation penetrates deeper into tissue, it may interact with more of the target physiological structures, potentially providing more comprehensive information. However, deeper penetration also results in greater attenuation of the optical radiation signal due to increased scattering and absorption. This weakened signal may be more difficult to detect and may have a lower signal-to-noise ratio, potentially reducing the accuracy of the measurement.

Conversely, optical radiation that does not penetrate as deeply may produce a stronger signal at the detector, but may not capture information from deeper tissue structures that could be relevant to the physiological parameter being measured.

For example, in the context of PPG, functional optical radiation contains pulsatile information from blood volume changes during the cardiac cycle. Photons with longer paths of optical radiation (i.e. `ray paths of optical radiation', or `ray paths') through pulsatile tissue may contain more pulsatile information contributing to cardiac cycle modulation in the sensor signal. Non-functional or "shunt" optical radiation in a pulse oximeter sensor does not contain pulsatile information and may introduce calibration artifacts in pulse oximeter and photoplethysmography (PPG) sensors.

Both reflection and transmission-based pulse oximetry configurations may be used. In transmission-based setups, the thickness of tissue like a finger determines the intensity of the detected signal at the photodiode. The tissue contains both pulsatile components (veins, arteries, capillaries) and non-pulsatile components (fat, bone, ligaments, nail). Ray paths of optical radiation may contain both functional and non-functional information and partially contribute to signal modulation depth. For reflection-based methods, increasing the distance between the LED and photodiode may increase photon penetration depth into tissue but decrease detected signal intensity. Deeper penetration may provide longer ray paths of optical radiation and larger modulation depth.

When applying sensors to thin tissue like the nostril or ear lobe, transmission-based pulse oximeter sensors may produce high detection signals but low modulation depth due to less pulsatile tissue involvement. For reflection-based pulse oximetry, the distance between optical source and photodiode may be critical to achieve sufficient penetration depth for larger modulation depth. In both cases, high detected signal and modulation depth may be desirable for high signal-to-noise ratio (SNR). Ray paths depend on various factors like distance, angle, and wavelength for both optical source and sensor.

### SUMMARY OF THE INVENTION

The invention is defined by the claims.

According to a first aspect of the invention, there is provided a system for measuring a biological signal of a subject. The system includes an optical unit configured to interface with a tissue of a subject. The system further includes a processing unit. The optical unit comprises one or more optical sources and one or more optical detectors. The processing unit is configured to perform a signal acquisition operation comprising controlling the optical unit to acquire a group of two or more optical signals from the tissue of the subject. The processing unit is further configured to compute a measurement of the biological signal from the group of optical signals. The processing unit is further configured to output the measurement of the biological signal. The optical signals of the group of optical signals may be acquired simultaneously. By this is meant that the optical signals of the groups of signals all span a common sampling time window. Each optical signal in the group corresponds to a different spatial ray path through the tissue of the subject from at least one of the one or more optical sources to at least one of the one or more optical detectors.

Thus, embodiments of the invention are based on the concept of acquiring optical signals across multiple ray paths through the tissue of the subject. This provides improved signal quality and improved measurement accuracy and reliability compared to single-path systems by capturing information from various tissue depths and along various trajectories through the tissue.

The invention leverages the idea that different ray paths through tissue can provide varying levels of signal quality for biological measurements. By simultaneously acquiring signals from multiple ray paths and selectively using or combining at least a subset of the signals (e.g. the highest quality signals in some embodiments), the system can potentially overcome challenges related to individual tissue variations and other sources of measurement error. These advantages could lead to more reliable and accurate optical biological signal measurements in clinical, consumer, and research applications, potentially improving patient care and expanding the utility of optical sensing technologies.

Acquiring the group of optical signals simultaneously means that the plurality of optical signals all span a common time window. Each optical signal may consist of a time series of sample points, wherein the sample points of each time series of each optical signal spans a common time window. Each optical signal may be generated by measurement of one or more optical projections through the tissue, the one or more optical projections following a ray path from at least one of the optical sources to the one of the optical detectors.

In some embodiments, the optical unit may comprise:
a plurality of optical sources and one or more optical detectors, or
one or more optical sources and a plurality of optical detectors,
which, in either case, are configured to define a plurality of different ray paths through the tissue of the subject when the optical unit interfaces with the tissue of the subject. This configuration enables the system to create multiple distinct ray paths through the tissue, increasing the likelihood of capturing high-quality signals and improving the overall measurement accuracy. This implementation effectively represents a static hardware implementation, wherein the multiple ray paths are achieved through the provision of a multiplicity of optical sources and/or detectors.

The alternative approach would be to use an optical unit having one or more moving sources or detectors, allowing for physical scanning of multiple ray paths.

In some embodiments, each optical signal in the group of signals may be associated with a quality metric indicative of a quality of the optical signal. In some embodiments, each optical signal in the group may be included in the group based on a value of the quality metric associated with the optical signal. The quality metric may be indicative of a quality of, or an estimated quality of, a biological signal contained in or carried by the optical signal. By utilizing quality metrics for each optical signal, the system can selectively use the highest quality signals for biological signal measurement, potentially improving the overall accuracy and reliability of the measurements. As will be discussed later, the system may be configured to perform a signal selection operation, where the group of optical signals is selected from a larger set of initially acquired signals based on evaluating a quality metric for each of the larger set of acquired optical signals.

Optionally, in some embodiments, the optical unit may comprise a plurality of optical sources and one or more optical detectors, wherein at least two of the plurality of optical sources are configured to emit optical radiation within different wavelength intervals. Using multiple wavelengths of optical radiation can provide additional information about the tissue and biological signals, potentially allowing for more comprehensive and accurate measurements of various physiological parameters. Optical signals having different wavelengths of optical radiation may contain different balances of functional vs non-functional optical radiation. The varying ratios of functional to non-functional optical radiation at different wavelengths adds another source of diversity to the set of optical signals used in computing the biological signal. This additional diversity may further improve accuracy and reliability of the measurements.

In some embodiments, the processing unit may be configured to perform a signal amalgamation operation which comprises amalgamating the group of optical signals to form a composite optical signal. The computing of the measurement of the biological signal from the group of optical signals may comprise computing the measurement of the biological signal from the composite signal. Amalgamating the optical signals into a composite signal improves the signal-to-noise ratio by combining information from multiple ray paths through the tissue. This approach can potentially enhance the overall quality of the biological signal measurement in several ways. By summing or averaging signals from different paths, noise may be reduced while the desired biological signal is reinforced. Additionally, combining signals that have traveled through different regions of the tissue may provide a more comprehensive sampling of the physiological information. The amalgamation process may also help mitigate the effects of localized artifacts or interference that may affect individual ray paths. Furthermore, by leveraging data from multiple paths, the system may be more robust to variations in tissue properties or sensor placement. Overall, signal amalgamation can potentially yield a composite signal with improved SNR compared to individual ray path signals, which may lead to more accurate and reliable measurements of biological parameters.

In some embodiments, the signal amalgamation operation may comprise summing the group of optical signals together and/or computing an average of the group of optical signals.

In some embodiments, the processing unit may be configured to perform a signal selection operation comprising: acquiring using the optical unit a set of optical signals, wherein the set of optical signals comprises a plurality of optical signals, each corresponding to a different ray path through the tissue of the subject, and preferably wherein the set of optical signals comprises a greater number of optical signals than is included in the group of optical signals. The signal selection operation may further comprise performing a quality metric determination operation comprising determining a quality metric for each of the plurality of optical signals in the set of optical signals. The signal selection operation may further comprise selecting a subset of the plurality of optical signals of the set of optical signals in dependence upon the quality metrics. The signal selection operation may further comprise identifying or recording the subset of the different ray paths to which the subset of the set of optical signals corresponds, and wherein the previously discussed group of optical signals corresponds to optical signals acquired using the subset of ray paths.

This signal selection process allows the system to dynamically choose the best ray paths for measurement, potentially adapting to variations in tissue properties or measurement conditions to maintain high-quality biological signal measurements.

After determining the quality metrics and selecting the subset of optical signals, the system may directly use those signals as the group of optical signals. Alternatively, the system may reacquire one or more times a new set of optical signals and select only those which correspond to the ray paths of the previously identified subset, discarding the rest and using that selection as the group of signals. In some embodiments, the system may physically acquire fewer signals after the signal selection operation, for example by only activating some of the optical sources and/or only sampling some of the detectors, if an arrangement with multiple sources and/or detectors is used.

In some embodiments, the quality metric determination operation may comprise, for each of the set of optical signals, detecting a biological signal in the optical signal, and estimating an SNR of the detected biological signal.

Using SNR as a quality metric can help ensure that the system selects the most informative and reliable optical signals for biological signal measurement.

In some embodiments, the quality metric determination operation may comprise applying a trained AI model, such as a convolutional neural network (CNN) or long short term memory (LSTM), to the set of optical signals.

The set of optical signals may correspond to a set of different ray paths, wherein the set of different ray paths includes ray paths that vary in ray path origin point and vary in ray path detection/termination point.

This variety in ray paths can provide a more comprehensive sampling of the tissue, potentially capturing biological signals from different tissue depths and structures.

The optical unit may comprise a tissue-facing area having an x-dimension and a y-dimension and wherein a plurality of optical sources are disposed at different X-Y locations across the tissue-facing area and/or a plurality of optical detectors are disposed at different X-Y locations across the tissue-facing area. This spatial arrangement of sources and detectors allows for a wide range of ray paths through the tissue.

The plurality of different ray paths may comprise ray paths of different directions, angles and/or ray path lengths through the tissue. Varying the angles/trajectories and lengths of ray paths can provide information from different tissue depths and structures, potentially improving the overall accuracy and comprehensiveness of the biological signal measurements.

The plurality of different ray paths may comprise ray paths having different origin location and/or different detection locations.

According to another aspect of the invention, there is provided a method of measuring a biological signal of a subject. The method comprises control of an optical unit configured to interface with a tissue of a subject, wherein the optical unit comprises one or more optical sources and one or more optical detectors. The method includes performing a signal acquisition operation comprising controlling the optical unit to acquire a group of optical signals from the tissue of the subject; computing a measurement of the biological signal from the group of optical signals; and outputting the measurement of the biological signal. The optical signals of the group of optical signals may be acquired simultaneously. This may mean that the group of optical signals all correspond to a common sampling time window. Each optical signal in the group corresponds to a different spatial ray path through the tissue of the subject from at least one of the one or more optical sources to at least one of the one or more optical radiation detectors.

In some embodiments, each optical signal in the group may be associated with a quality metric indicative of a quality of an optical signal, wherein each optical signal in the group is included in the group based on a value of the quality metric associated with the optical signal. By incorporating quality metrics, the method can selectively use the highest quality signals for biological signal measurement, potentially improving the overall accuracy and reliability of the measurements.

According to another aspect of the invention, there is provided a computer program product comprising computer program code configured, when run by a computer, to perform a method in accordance with any embodiment or example detailed in this disclosure or in accordance with any claim.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a block diagram of a system for measuring biological signals, according to one or more embodiments of the invention;
Fig. 2 shows a cross-sectional view of an optical unit interfacing with tissue, in accordance with one or more embodiments;
Fig. 3 depicts a functional block diagram of signal processing in the system of Fig. 1, in accordance with one or more embodiments;
Fig. 4A and Fig. 4B illustrate a variant of the system of Fig. 1, in which a transmission measurement approach is used instead of a reflection measurement approach;
Fig. 5 presents a cross-sectional view of the system of Fig. 4A and Fig. 4B;
Fig. 6 illustrates a block diagram of steps for signal amalgamation in accordance with one or more embodiments;
Fig. 7 provides a visual representation of an example group of optical signals over time;
Fig. 8 illustrates steps of an example signal selection operation in accordance with one or more embodiments; and
Fig. 9 illustrates steps of an example process for evaluating a quality metric for acquired optical signals.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for measuring biological signals, such as oxygen saturation levels in the blood, using optical sensors. The method is based on computing a biological signal using a plurality of optical signals, the plurality of optical signals acquired across multiple ray paths through the tissue. In some embodiments, the optical signals of the group are acquired simultaneously, and each optical signal in the group corresponds to a different spatial ray path through the tissue of the subject from at least one optical source to at least one optical detector.

In some cases, each optical signal in the group may be associated with a quality metric indicative of the quality of the optical signal. Each optical signal in the group may be included in the group based on a value of the quality metric associated with the optical signal. By incorporating quality metrics for each optical signal, the system can selectively use the highest quality signals for biological signal measurement, potentially improving the overall accuracy and reliability of the measurements.

Embodiments of the present invention thus provide a system and method for measuring biological signals that may offer improved signal-to-noise ratio and thus enhanced measurement accuracy and reliability through dynamic selection of optimal ray paths.

The acquisition of optical signals across multiple ray paths provides a means for optimizing a balance between penetration depth and signal strength in optical biological signal measurement systems. This strategy could be understood as a form of spatial diversity of signal acquisition, a technique in which system performance may be improved in the presence of uncertainties or variations in the signal propagation environment. In effect, multiple copies of the same signal are received through different channels and then combined to improve overall signal quality.

For example, by simultaneously acquiring signals from multiple ray paths, the system may inherently incorporate redundancy that allows for the extraction of maximal biological signal information without the need for a priori knowledge of the optimal ray paths. This approach may be particularly advantageous in scenarios where tissue characteristics vary among individuals or change over time. The system may acquire signals from a diverse set of ray paths and then potentially employ post-acquisition processing to identify and utilize the most informative signals, or simply combine the signals together to maximize the SNR.

In the context of optical biological signal measurement, each ray path may be understood as representing a different signal channel, potentially providing unique information about the underlying physiological parameters. By acquiring and processing signals from these multiple channels, the system may effectively implement a form of spatial diversity that can enhance measurement robustness and accuracy.

One aspect of the invention is a system for measuring a biological signal of a subject. Another aspect of the invention is a method of measuring a biological signal of a subject. Another aspect of the invention is a computer program product comprising computer program code configured, when run on a computer, to cause the computer to perform a method in accordance with any example or embodiment detailed in this disclosure or in accordance with any claim.

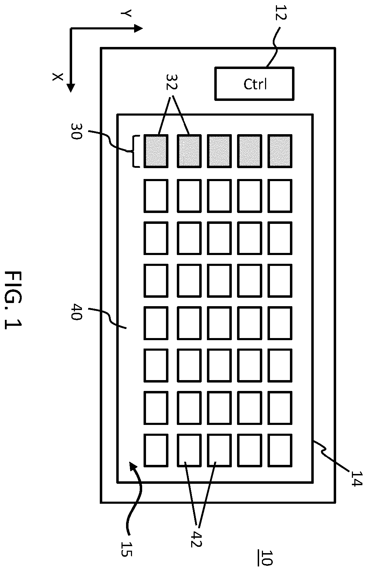

An example implementation of a system which is in accordance with one or more embodiments of the invention will now be discussed, with reference to Fig. 1, Fig. 2 and Fig. 3.

Fig. 1 schematically illustrates the example system 10.

The system 10 is for measuring a biological signal of a subject.

The system 10 includes an optical unit 14. The optical unit comprises a set 30 of one or more optical sources 32. The optical unit further comprises a set 40 of one or more optical detectors 42. The optical unit 14 is configured to interface with a tissue 52 of a subject.

In some embodiments, each optical source 32 may comprise one or more light-emitting diodes (LEDs) emitting optical radiation (e.g. light) at specific wavelengths. In some embodiments, each optical detector 42 may include one or more photodiodes, phototransistors, or other photodetectors sensitive to the wavelengths emitted by the optical sources.

The optical unit 14 may comprise a tissue-facing area 15, wherein the tissue facing area is for optically coupling with a tissue 52 surface during operation. It may be for physically engaging with the tissue surface during use. The one or more optical sources 32 are arranged such that an optical output of each optical source is optically coupled with the tissue when the tissue-facing area is interfacing with the tissue of the subject. The one or more optical detectors 42 are arranged such that an optical input area of each optical source is optically coupled with the tissue when the tissue-facing area is interfacing with the tissue of the subject.

The system 10 further comprises a processing unit 12.

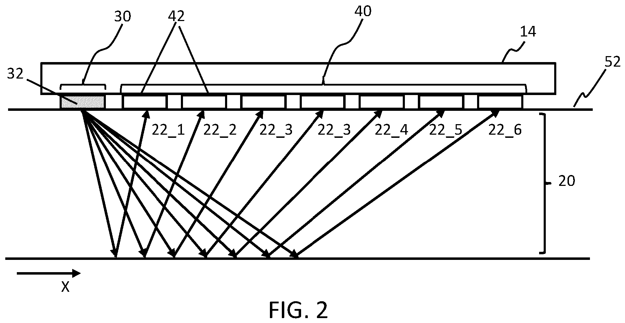

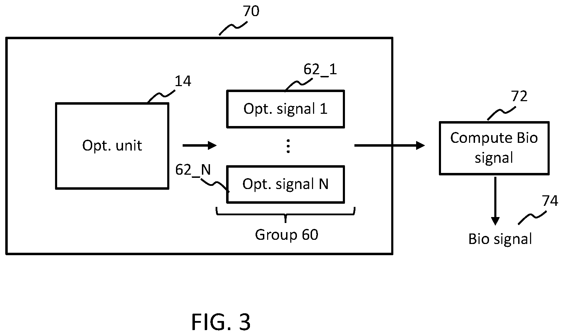

With reference to Fig. 2 and Fig. 3, the processing unit 12 is configured to perform a signal acquisition operation 70. The signal acquisition operation 70 comprises controlling the optical unit 14 to acquire a group 60 of two or more optical signals 62_1,... ,62_N from the tissue 52 of the subject. Each optical signal is measured at one of the one or more optical detectors. A single optical signal may be referred to generically in this disclosure with the reference numeral 62_n.

With reference to Fig. 2 and Fig. 3, the optical unit is configured, and the signal acquisition operation is configured, such that each optical signal 62_n in the group 60 of acquired optical signals corresponds to a different spatial ray path 22_n through the tissue 52 of the subject from at least one of the one or more optical sources 32 to at least one of the one or more optical detectors 42. Each optical signal 62_n may be formed by measurement of one or more optical projections through the tissue, the one or more optical projections following an ray path 22_n from at least one of the optical sources 32 to at least one of the optical detectors 42. The optical projections may follow ray paths from one or more of the optical sources 32 to one or more of the optical detectors 42.

According to one non-limiting set of embodiments, the optical signals may be PPG signals.

The processing unit 12 is further configured to compute 72 a measurement of the biological signal 74 using the group 60 of optical signals 62_n. In some embodiments, this may comprise applying a biological signal computation algorithm which receives one or more inputs and generates as an output one or more measurement values of the biological signal 74. The one or more inputs may be derived from the group of optical signals. In some cases, the inputs may be the set of optical signals themselves, or may be a composite signal computed from the group of optical signals, or may be different information inputs derived from the group of optical signals. By way of one non-limiting example, the biological signal may be a vital sign.

The processing unit 12 may be further configured to output the measurement of the biological signal 74.

The signal acquisition operation 70 may be configured such that the optical signals 62_n of the group 60 of optical signals all span a common acquisition time window. In other words, the group of optical signals are acquired temporally in parallel. In other words, the group of optical signals are acquired simultaneously in time. For example, each optical signal of the group of optical signals may consist of a time series of sample points, and wherein the time series forming each optical signal of the group of optical signals spans the same time window.

Referring to Fig. 2, a schematic cross-section of an example optical unit 14 is depicted. The optical unit 14 is positioned above a tissue 52 to be measured. The optical unit 14 comprises a set 30 of one or more optical sources 32 and a set 40 of one or more optical detectors 40. In the particular example depicted in Fig. 1 and Fig. 2, the set 30 of optical sources 32 comprises a plurality of optical sources and the set 40 of optical detectors 42 comprises a plurality of optical detectors. This configuration enables the optical unit to acquire optical signals across multiple distinct ray paths through the tissue 52 in a static state configuration, without any need for moving parts. However, an alternative approach would be to provide a moving optical source and/or a moving optical detector, allowing for acquisition of optical signals across multiple spatial paths through the tissue by moving or scanning one or both of the optical source and optical detector across multiple positions (or angles transferred into positions in case of sources placed at certain position from skin).

Acquisition of each optical signal 62_n comprises one of the set 30 of one or more optical sources 32 generating an optical emission which is transmitted into the tissue 52 and one of the set 40 of optical detectors 42 detecting the optical emission after it has passed through the tissue 52 along a particular spatial ray path 22_n through the tissue 52.

Fig. 2 schematically illustrates a set 20 of six spatial ray paths 22_1, 22_2, 22_3, 22_4, 22_5, 22_6 through the tissue. However, this is for purposes of illustration, and a practical embodiment may acquire optical signals across a greater number or smaller number of ray paths 22_n. A single ray path may be referred to generically in this disclosure using the reference numeral 22_n. Each ray path 22_n originates from one of the one or more optical sources 32 and terminates at one of the one or more optical detectors 42 after passing through the tissue 52. The ray paths 22_n vary in angle and length, providing different sampling depths within the tissue 52. This arrangement allows for the simultaneous acquisition of multiple optical signals corresponding to different spatial paths through the tissue.

The schematic depiction in Fig. 2 shows a set of ray paths 22_n which all originate from the same single optical source 32 and which terminate at respectively different optical detectors 42. However, in alternative arrangements, multiple ray paths might originate from multiple different respective optical sources 32 and terminate at a same optical detector. Likewise, different ray paths may differ from one another both in terms of their origin point and their termination points.

In the example arrangement shown in Fig. 1 and Fig. 2, the set 30 of one or more optical sources 32 comprises a linear 1D array of optical sources. However, this is only an example. In further examples, the set 30 of one or more optical sources 32 may comprise a 2D array of optical sources, and/or may comprise an array of optical sources of a different geometry or shape, and/or may consist of a single optical source.

In the example arrangement shown in Fig. 1 and Fig. 2, the set 40 of one or more optical detectors 42 comprises a 2D array of optical detectors. However, this is only an example. In further examples, the set 40 of one or more optical detectors 42 may comprise a 2D array of optical detectors, and/or may comprise an array of optical detectors of a different geometry or shape, and/or may consist of a single optical detector.

In some embodiments, the set 40 of one or more optical detectors 42 may be implemented with a camera, and wherein different optical detectors 42 correspond to different pixels across the field of view of the camera.

In the example of Fig. 1 and Fig. 2, the optical unit 14 includes a tissue-facing area 15 with an x-dimension and a y-dimension. In some cases, different optical sources 32 of the set 30 of different optical sources are disposed at different X-Y locations across the tissue-facing area 15. Alternatively or additionally, different optical detectors 42 of the set 40 of optical detectors 42 may be disposed at different X-Y locations across the tissue-facing area 15. This arrangement of optical sources 32 and optical detectors 42 allows for the creation of multiple distinct ray paths through the tissue 52 in an efficient way.

The spacing and arrangement of the one or more optical sources 32 and one or more optical detectors 42 across the tissue-facing area 52 may vary based on the specific application and form factor. In some embodiments, the sources and detectors may be arranged in a grid pattern with spacing of a few millimeters to a few centimeters between components.

Acquiring an optical signal from the tissue may involve activating one or more optical sources 32 to emit optical radiation into the tissue and detecting the transmitted or reflected optical radiation using one or more optical detectors 42. The detected optical radiation intensity (e.g. optical radiation received by an optical sensor) over time forms the optical signal. The control of the optical unit to acquire a group of two or more optical signals may comprise activating multiple optical sources simultaneously or in a cyclical sequence pattern and sampling the one or more optical detectors to capture the emitted optical radiation after passing through the tissue. The processing unit 12 may be configured to synchronize the timing of source activation and detector sampling so as to capture an optical detection of each individual one of the group of different optical signals. The processing unit 12 may be configured to digitize and record the intensity readings from each detector over time. The processing unit may be configured to associate each stored signal with metadata about its corresponding ray path.

In some embodiments, the system 10 may employ a time multiplexing approach to acquire a plurality of optical signals 62_n simultaneously, where the term 'simultaneously' refers to all signals spanning the same time window, but with time-interleaved sampling of the sample points for each optical signal. This approach may allow for efficient use of hardware resources while still capturing information from multiple ray paths within a common timeframe. It may also enable simultaneous measurements in a specific time window, as the resolution and speed of sampling in relation to the relatively slow signal to measure allows for a time multiplexing approach.

The time multiplexing approach may involve rapidly switching between different optical sources and detectors to sample multiple ray paths 22_n in quick succession. This process may be repeated at a high frequency to create the effect of simultaneous acquisition across all paths. The processing unit 12 may control this multiplexing process through precise timing and synchronization of the optical sources and detectors.

In some implementations, the system may use a multiplexing circuit or code (e.g. pseudorandom noise (PRN) or gold code) to control the activation of optical sources 32 and the sampling of optical detectors 42. This circuit/code may be configured to cycle through a predetermined sequence of source-detector combinations, with each combination corresponding to a specific ray path 22_n through the tissue 52.

The multiplexing sequence may be designed to optimize the sampling of each ray path 22_n while minimizing potential interference between paths. For example, the system may alternate between spatially distant ray paths to reduce crosstalk between adjacent paths.

The sampling rate for each individual ray path 22_n may be determined based on factors such as the desired overall sampling frequency, the number of ray paths being measured, and the characteristics of the biological signal being monitored.

To reconstruct the optical signals for each ray path 22_n, the processing unit may use demultiplexing techniques to separate the interleaved samples into a distinct time series for each ray path, and the time series for each ray path forming a respective optical signal 62_n. By way of example, this process may involve sorting the samples based on their associated metadata or timestamp, which may for example include information about the specific source-detector combination used for each sample.

In some embodiments, the system may employ interpolation techniques to estimate optical signal values between the actual sampled points for each ray path. This may help to create a more continuous representation of each optical signal, despite the time-multiplexed sampling approach.

The example system depicted in Fig. 1 and Fig. 2 represents a reflection-type optical system, wherein the optical sources 32 and optical detectors 42 are arranged on a same side of the tissue being probed and wherein the optical signals are detected after reflection of optical radiation emitted by the optical sources 32 from the tissue 52. However, as will be known to the skilled person, in the field of optical measurement of biological signals, also transmission-type systems are possible.

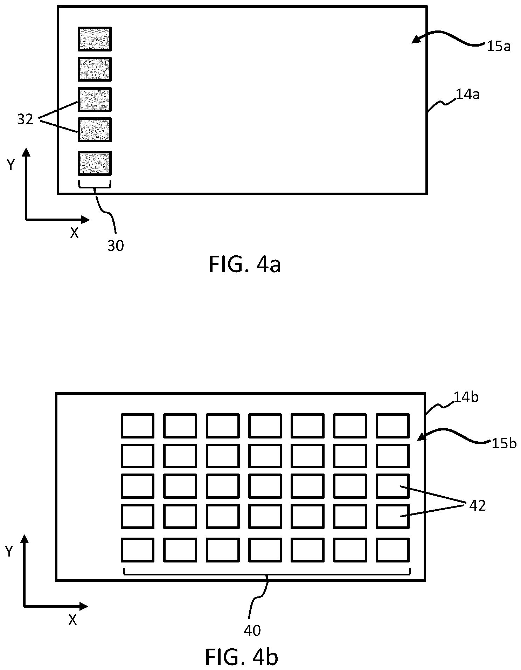

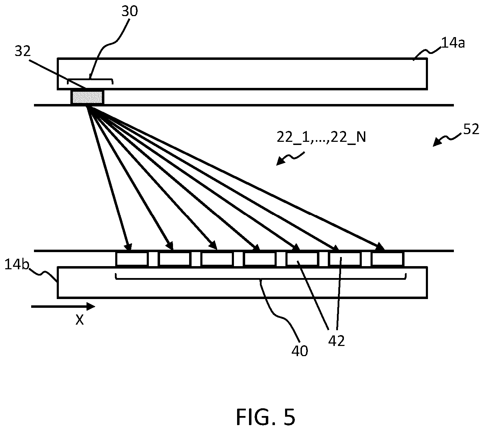

Fig. 4A, Fig. 4B, and Fig. 5 schematically illustrate a variant of the system represented in Fig. 1, Fig. 2 and Fig. 3 in which the optical signals are generated on one side of the subject's tissue 52 and detected on an opposite side of the tissue, after passing through the tissue. Thus, in this example, the optical unit 14 has two parts. The optical unit comprises a first part 14a for placement during use on one side of a tissue of the subject and a second part 14b for placement during use on an opposite side of the tissue of the subject. In this example, the first part 14a carries the set 30 of one or more optical sources 32 and the second part 14b carries the set 40 of one or more optical detectors 42. However, in further examples, the first part 14a and the second part 14b may each carry a subset of the set 30 of optical sources and a subset of the set 40 of optical detectors. In this variant, the tissue facing area has two parts: a first tissue facing area part 15a arranged facing the tissue from one side of the tissue and a second tissue facing area part 15b arranged facing the tissue from the opposite side of the tissue. The first 15a and second 15b parts of the tissue-facing area are in optical communication via the tissue 52 of the subject. Each of the first 15a and second 15b parts of the tissue-facing area may have an x-dimension and a y-dimension. In some cases, different optical sources 32 of the set 30 of different optical sources are disposed at different X-Y locations across the first part 15a of the tissue-facing area. Alternatively or additionally, different optical detectors 42 of the set 40 of optical detectors 42 may be disposed at different X-Y locations across the second part 15b of the tissue-facing area.

In all other respects the example system 10 of Fig. 4A, Fig. 4B, and Fig. 5 may be the same as the example system of Fig. 1, Fig. 2 and Fig. 3.

In the examples of Figs. 1-5, the optical unit 14 comprises a plurality of optical sources 32 and one or more optical detectors 42, or comprises one or more optical sources 32 and a plurality of optical detectors 42, which are together configured to define a plurality of different ray paths 22_1,...,22_N through the tissue 52 of the subject when the optical unit 14 interfaces with the tissue of the subject.

However, a variant is also possible in which, at minimum, the optical unit 14 comprises a single optical source 32 and single optical detector 42 and wherein at least one of the optical source and optical detector are configured to move to scan a plurality of different physical ray paths.

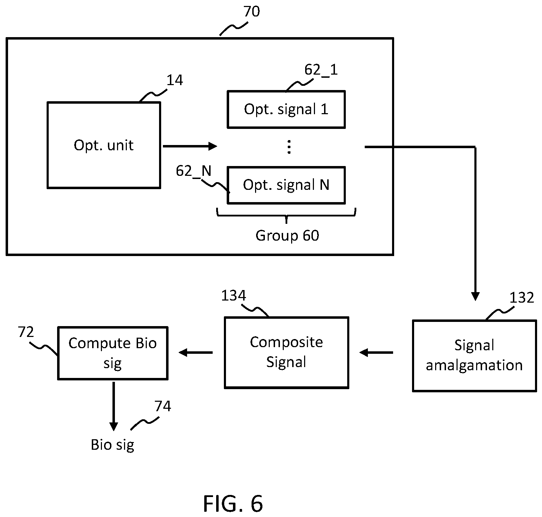

With reference to Fig. 6, in some embodiments, the processing unit 12 is configured to perform a signal amalgamation operation 132. The signal amalgamation operation may be performed following the signal acquisition operation 70 previously discussed with reference to Fig. 3. The signal amalgamation operation may comprise amalgamating the group 60 of optical signals 62_n (which are the output of the signal acquisition operation 70) to form a composite optical signal 134. The computing 72 of the measurement of the biological signal 74 from the group 60 of optical signals 62_n may comprise computing the measurement of the biological signal 74 from the composite signal 134.

By way of example, the signal amalgamation operation 132 may comprise summing the group 60 of optical signals 62_n together. In this approach, the group 60 of optical signals 62_n are added together. This method can enhance the overall signal strength, potentially improving the signal-to-noise ratio. However, it may also amplify any common noise present in all signals. By way of further example, the signal amalgamation operation 132 may comprise averaging the group 60 of optical signals 62_n. This approach involves computing the mean of the group 60 of optical signals 62_n. Averaging can help reduce noise in the signals, as noise tends to cancel out when multiple signals are averaged, while the consistent biological signal is reinforced. The average may be a weighted average in some embodiments.

By combining/amalgamating multiple signals acquired across different ray paths, wherein all the signals correspond to a same sampling time period, it is possible to improve SNR and improve reliability of the computed biological signal.

As noted above, the group 60 of optical signals 62_n corresponds to a set of different ray paths 22_n, wherein the set of different ray paths may include ray paths which vary in their ray path origin points and/or vary in their ray path detection/termination points. The origin and detection points may each vary across an X-Y plane, so that there are effectively four degrees of freedom in varying the ray paths: X-Y location of the ray path origin and X-Y location of the ray path detection point. This provides a large degree of possible variation in ray path trajectory through the tissue.

In more detail, as noted previously, in some embodiments the optical unit 14 comprises a tissue-facing area 15 having an x-dimension and a y-dimension and wherein a plurality of optical sources 32 are disposed at different X-Y locations across the tissue-facing area and/or a plurality of optical detectors 42 are disposed at different X-Y locations across the tissue-facing area. The group 60 of optical signals 62_n may correspond to a group of ray paths 22_n which vary in their X-Y origin and/or X-Y termination points. The plurality of different ray paths may comprise ray paths of different angles and/or ray path lengths through the tissue 52.

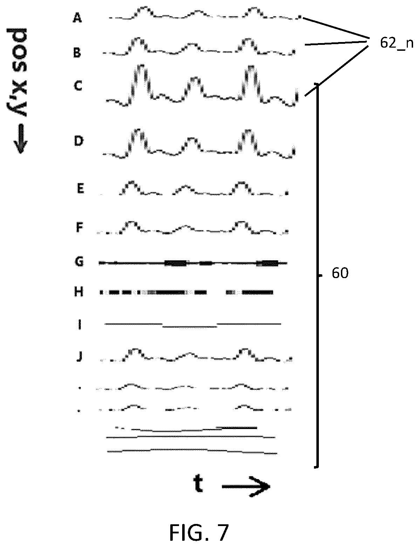

The group of optical signals 62_n may be summed across the time-axis. This is illustrated schematically in Fig. 7 which shows an example group 60 of different optical signals 62_n aligned along the time axis (t). The set of different optical signals differ with respect to the X-Y location of their respective source points and/or detection points, but all span a same time window across the time dimension (t). It can be observed that in some of the signals, the biological signal (pulse rate in this case) is clearly visible, while in others the signal is weak or even absent. By combining the signals from the different ray paths, the vital sign signal is the sum of all the signals present across the different optical radiation paths, resulting in an enhanced vital sign signal as compared against the noise.

The group 60 of optical signals 62_n may be aligned or synchronized in the time domain prior to the signal amalgamation operation 132 in some embodiments. For example, the signals may be phase locked according to a detected phase of the biological signal (e.g. vital sign) present in the optical signal.

In some embodiments, if the optical unit 14 comprises a plurality of optical sources 32 and one or more optical detectors 42, at least two of the plurality of optical sources 32 may be configured to emit optical radiation within different wavelength intervals. This adds an additional degree of diversity to the different optical signals which may further enhance the reliability of the measured biological signal.

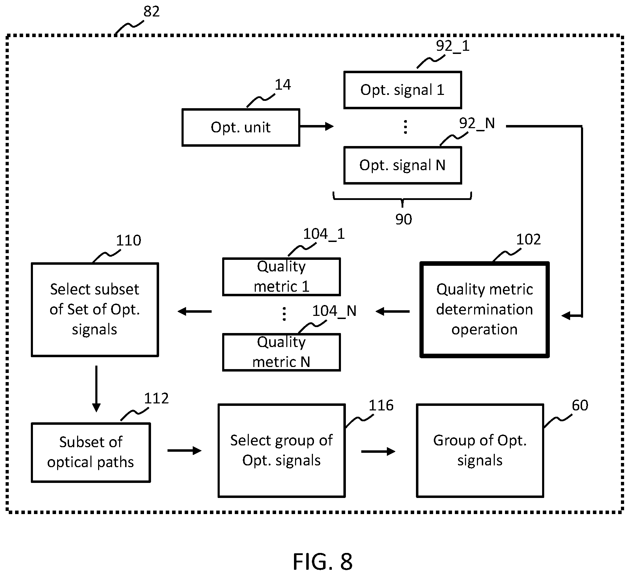

With reference to Fig. 8, in some embodiments the processing unit 12 is configured to perform a signal selection operation 82. The signal selection operation 82 comprises acquiring using the optical unit 14 a set 90 of optical signals 92_n. The set 90 of optical signals comprises a plurality of optical signals 92_n, each corresponding to a different ray path 22_n through the tissue 52 of the subject. Preferably, the acquired set 90 of optical signals 92_n comprises a greater number of optical signals than is included in the previously discussed group 60 of optical signals. The processing unit may be further configured to perform a quality metric determination operation 102 comprising determining a respective quality metric 104_1,...,104_N for each of the plurality of optical signals 92_n in the set 90 of optical signals. In this disclosure a single quality metric may be referred to generically by the reference numeral 104_n. The processing unit may be further configured to select 110 a subset of the plurality of optical signals 92_n of the set 90 of optical signals in dependence upon the quality metrics 104_n. The processing unit may be further configured to identify the subset 112 of the different ray paths 22_n to which the subset of the set 90 of optical signals 92_n correspond. The previously mentioned group 60 of optical signals 62_n corresponds to optical signals acquired using the subset of ray paths. In other words, in this set of embodiments, the signal selection operation further comprises selecting 116 the optical signals to be included in the group 60 of optical signals, wherein the optical signals selected for inclusion in the group are the optical signals acquired using the identified subset 112 of ray paths.

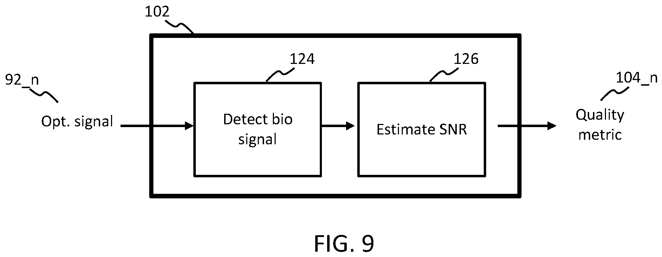

With regards to the quality metric determination operation 102, and with reference to Fig. 9, this may comprise, for each of the set 90 of optical signals 92_n, detecting 124 a biological signal in the optical signal, and estimating 126 an SNR of the detected biological signal. Detecting the biological signal in the optical signal may involve analyzing the optical signal 92_n to identify characteristics indicative of physiological processes, such as pulsatile blood flow. This may be accomplished through techniques such as filtering, peak detection, or frequency analysis. Estimating the SNR of the detected biological signal may involve comparing the amplitude or power of the detected biological signal to the background noise level in the optical signal. This could be done by calculating the ratio of signal power to noise power in relevant frequency bands. In some embodiments, more advanced signal processing techniques such as adaptive filtering or wavelet analysis may be used to improve signal detection and SNR estimation. The specific methods used may depend on factors such as the type of biological signal being measured and the characteristics of the optical sensing system.

In some embodiments, the quality metric determination operation 102 may be implemented using a trained artificial intelligence (AI) model, such as a convolutional neural network (CNN) or LSTM (or combination thereof). The CNN may be configured to analyze the optical signals and determine quality metrics for each signal.

To implement this embodiment, the system may utilize a CNN architecture designed for time series analysis. The CNN may include multiple convolutional layers followed by pooling layers and fully connected layers. The input to the CNN may be a segment of the optical signal 92_n, such as a 5-10 second window. Also, the signal(s) may be converted to an image presentation of the graph so it fits into an image based CNN model.
The output may be a quality metric 104_n value between 0 and 1, with higher values indicating higher quality signals.

The CNN may be trained on a large dataset of labeled optical signals. The training data may include signals with known values for the quality metric 104_n, ranging from high-quality signals with clear physiological information to low-quality signals. The training process may involve optimizing the CNN parameters to minimize the difference between the predicted quality metrics and the ground truth labels.

During operation, the trained CNN may process each optical signal in the set 90 of optical signals 92_n. The CNN may extract relevant features from the time series data, such as the presence and strength of physiological rhythms, signal-to-noise ratio, and stability of the baseline. Based on these extracted features, the CNN may output a quality metric 104_n for each signal.

Optionally, following the signal selection operation 82, the processing unit 12 is configured to perform a measurement operation comprising recurrently acquiring a measurement set of the group 60 of optical signals 62_n and computing 72 a respective value of the biological signal 74 based on each acquired measurement set of the group 60 of optical signals. In other words, in certain embodiments, the system may perform the signal selection operation 82 once to identify high-quality ray paths and subsequently, the system may recurrently acquire the group 60 of signals corresponding only to the highest quality ray paths identified.

However, another approach is to perform a more real-time continuous quality assessment, for example wherein the signal selection operation 82 is performed each time that optical signal measurements are acquired. In this way, the selected group of optical signals may change dynamically over time over the course of a measurement operation.

In some cases, the system may employ a hybrid approach, regularly acquiring signals from a subset of pre-selected high-quality paths while periodically sampling additional paths to detect potential improvements in signal quality. This may allow the system to balance efficient operation with the ability to adapt to changing conditions.

With regards to the computation of a biological signal 74 from the acquired optical signals 62_n, different options are possible.

In some embodiments, the optical signals 62_n acquired by the system may be photoplethysmography (PPG) signals, and the biological signal 74 may be a vital sign. PPG is a non-invasive optical technique that can detect blood volume changes in the microvascular bed of tissue 52. The PPG waveform comprises a pulsatile ('AC') physiological waveform attributed to cardiac synchronous changes in the blood volume with each heart beat, and a slowly varying ('DC') baseline with various lower frequency components attributed to respiration, sympathetic nervous system activity and thermoregulation.

In these embodiments, the optical unit 14 may emit optical radiation into the tissue 52 and measure the amount of optical radiation that is absorbed or reflected. The amount of optical radiation absorbed or reflected may change with the pulse of blood flow. This pulsatile component of the PPG signal may be used to derive various vital signs.

For example, the vital sign may be blood oxygen saturation (SpO2). In this case, the optical unit 14 may include at least two optical sources 32 emitting optical radiation at different wavelengths, typically red (around 660 nm) and infrared (around 880 to 940 nm). The optical detectors 42 may measure the amount of optical radiation transmitted through or reflected from the tissue at each wavelength. In some embodiments, a respective group 60 of optical signals 62_n, corresponding to a group of different ray paths 22_n, may be acquired for each of the two wavelengths.

The processing unit 12 may analyze the PPG signals obtained from the different ray paths to compute the blood oxygen saturation. The computation may be based on the ratio of absorbance of red and infrared optical radiation, which varies with the oxygen saturation of hemoglobin in the blood.

The processing unit 12 may employ various algorithms to calculate the blood oxygen saturation, such as the ratio-of-ratios method or more advanced techniques like adaptive filters or machine learning models. These algorithms are designed to account for individual variations in tissue properties and potential artifacts in the signal.
By using a composite PPG signal derived from optical signals 62_n acquired across multiple ray paths through the tissue, the system can potentially achieve more robust and accurate SpO2 measurements compared to traditional single-path systems.

In some cases, the system 10 may provide continuous monitoring of blood oxygen saturation. The signal selection operation 82 may be repeated periodically.

In addition to blood oxygen saturation, the PPG signals acquired through multiple ray paths may be used to derive other vital signs. These may include heart rate, which can be determined from the frequency of the pulsatile component of the PPG signal, and respiration rate, which may be extracted from lower frequency variations in the PPG baseline or amplitude.

It is noted that, for pulse oximetry sensors with multiple wavelengths of optical radiation, the principles of the invention can still be applied. In this case, by way of example, a respective group 60 of optical signals 62_n corresponding to different ray paths 22_n through the tissue 52 may be acquired for each of the two or more wavelengths of optical radiation and the biological signal can be computed using the groups of optical signals. A respective signal amalgamation operation 132 may optionally be applied for each wavelength of optical radiation. A respective signal selection operation 82 may optionally be applied for each wavelength of optical radiation.

Although an example has been discussed above in which the measured biological signal is blood oxygen saturation, the principles of the invention may be applied to measure a wide range of biological signals. By way of non-limiting example, these may include one or more vital signs such as heart rate, respiration rate, blood pressure, and glucose levels.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs). For instance, sources and detectors can be integrated on-chip, including electronics, as Photonic Integrated Circuits (PICs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A system (10) for measuring a biological signal (74) of a subject, the system comprising:
an optical unit (14) configured to interface with a tissue (52) of a subject; and
a processing unit (12);
wherein the optical unit (14) comprises one or more optical sources (32) and one or more optical detectors (42); and
wherein the processing unit (12) is configured to:
perform a signal acquisition operation (70) comprising controlling the optical unit (14) to acquire a group (60) of two or more optical signals (62_n) from the tissue (52) of the subject; and
compute (72) a measurement of the biological signal (74) from the group (60) of optical signals (62_n); and
output the measurement of the biological signal (74);
wherein the optical signals (62_n) of the group (60) of optical signals are acquired simultaneously; and
wherein each optical signal (62_n) in the group (60) corresponds to a different spatial ray path (22_n) through the tissue (52) of the subject from at least one of the one or more optical sources to at least one of the one or more optical detectors.

2. The system of claim 1,
wherein the optical unit (14) comprises a plurality of optical sources (32) and one or more optical detectors (42), or one or more optical sources (32) and a plurality of optical detectors (42), which are configured to define a plurality of different ray paths (22_1, ... ,22_N) through the tissue (52) of the subject when the optical unit (14) interfaces with the tissue of the subject.

3. The system of claim 1,
wherein each optical signal (62_n) in the group (60) is associated with a quality metric (104_n) indicative of a quality of the optical signal (62_n);
wherein each optical signal (62_n) in the group (60) is included in the group (60) based on a value of the quality metric (104_n) associated with the optical signal (62_n).

4. The system (10) of any preceding claim,
wherein the optical unit (14) comprises a plurality of optical sources (32) and one or more optical detectors (42); and
wherein at least two of the plurality of optical sources (32) are configured to emit optical radiation within different wavelength intervals.

5. The system (10) of any preceding claim,
wherein the processing unit (12) is configured to perform a signal amalgamation operation (132) which comprises amalgamating the group (60) of optical signals (62_n) to form a composite optical signal (134); and
wherein the computing (72) the measurement of the biological signal (74) from the group (60) of optical signals (62_n) comprises computing the measurement of the biological signal (74) from the composite signal (134).

6. The system (10) of claim 5, wherein the signal amalgamation operation (132) comprises summing the group (60) of optical signals (62_n) together and/or comprises computing an average of the group (60) of optical signals (62_n).

7. The system (10) of any preceding claim, wherein the processing unit (12) is configured to perform a signal selection operation (82) comprising:
acquiring using the optical unit a set 90 of optical signals (92_n), wherein the set (90) of optical signals comprises a plurality of optical signals (92_n), each corresponding to a different ray path (22_n) through the tissue (52) of the subject;
wherein the set (90) of optical signals (92_n) comprises a greater number of optical signals than is included in the group (60) of optical signals;
performing a quality metric determination operation (102) comprising determining a quality metric (104_n) for each of the plurality of optical signals (92_n) in the set (90) of optical signals;
selecting (110) a subset (112) of the plurality of optical signals (92_n) of the set (90) of optical signals in dependence upon the quality metrics (104_n);
identifying the subset (112) of the different ray paths (22_n) to which the subset of the set (90) of optical signals (92_n) correspond; and
wherein the group (60) of optical signals (62_n) corresponds to optical signals acquired using the subset of ray paths.

8. The system of claim 7, wherein the quality metric determination operation (102) comprises, for each of the set (90) of optical signals (92_n), detecting (124) a biological signal in the optical signal, and estimating an SNR of the detected biological signal.

9. The system of claim 7 or 8, wherein the set of optical signals corresponds to a set of different ray paths, wherein the set of different ray paths includes ray paths with vary in ray path origin point and vary in ray path detection/termination point.

10. The system (10) of any of claims 2-9,
wherein the optical unit (14) comprises a plurality of optical sources (32) and one or more optical detectors (42), or one or more optical sources (32) and a plurality of optical detectors (42), which are configured to define a plurality of different ray paths (22_1, ... ,22_N) through the tissue (52) of the subject when the optical unit (14) interfaces with the tissue of the subject; and
wherein the optical unit (14) comprises a tissue-facing area (15) having an x-dimension and a y-dimension and wherein a plurality of optical sources (32) are disposed at different X-Y locations across the tissue-facing area and/or a plurality of optical detectors (42) are disposed at different X-Y locations across the tissue-facing area.

11. The system of any preceding claim, wherein the plurality of different ray paths (22_n) comprise ray paths of different angles/trajectories and/or ray path lengths through the tissue.

12. A method of measuring a biological signal (74) of a subject,
the method comprising control of an optical unit (14) configured to interface with a tissue (52) of a subject, wherein the optical unit (14) comprises one or more optical sources (32) and one or more optical detectors (42); and
wherein the method comprises:
performing a signal acquisition operation (70) comprising controlling the optical unit to acquire a group (60) of optical signals (62_n) from the tissue (52) of the subject;
computing a measurement of the biological signal (74) from the group (60) of optical signals (62_n); and
outputting the measurement of the biological signal (74);
wherein the optical signals (62_n) of the group (60) of optical signals are acquired simultaneously;
wherein each optical signal (62_n) in the group (60) corresponds to a different spatial ray path (22_n) through the tissue (52) of the subject from at least one of the one or more optical sources to at least one of the one or more optical detectors.

13. The method of claim 12,
wherein each optical signal (62_n) in the group (60) is associated with a quality metric (104_n) indicative of a quality of an optical signal (62_n);
wherein each optical signal (62_n) in the group (60) is included in the group (60) based on a value of the quality metric (104_n) associated with the optical signal (62_n).

14. A computer program product comprising computer program code configured, when run by a computer, to a perform a method in accordance with claim 12 or 13.
